(19) Europäisches Patentamt
European Patent Office
Office européen des brevets

(11) **EP 3 136 343 A1**

(12) **EUROPEAN PATENT APPLICATION**

(43) Date of publication:
**01.03.2017 Bulletin 2017/09**

(51) Int Cl.:
**G06T 7/00** (2017.01)

(21) Application number: **16185927.7**

(22) Date of filing: **26.08.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **28.08.2015 GB 201515395**

(71) Applicant: **Perspectum Diagnostics Ltd**
**New Road, Oxford Oxfordshire OX1 1BY (GB)**

(72) Inventors:
• **KELLY, Catherine**
  **Oxford, Oxfordshire OX1 1BY (GB)**
• **VIKAL, Siddharth**
  **Oxford, OX1 1BY (GB)**
• **HARAMIJA, Marija**
  **Oxford, Oxfordshire OX1 1BY (GB)**

(74) Representative: **Optimus Patents Ltd**
**Grove House, Lutyens Close**
**Chineham Court**
**Basingstoke Hampshire RG24 8AG (GB)**

(54) **A METHOD AND APPARATUS FOR GENERATING A DATA PROFILE FOR A MEDICAL SCAN**

(57) A method and apparatus for generating a data profile for a medical scan. The method comprises obtaining data point values for a plurality of data points representing spatial locations within the medical scan, determining data point classification parameters defining data point value ranges for a plurality of classes, assigning each data point to a class having a data point value range corresponding to the value for that data point, generating a data profile for the medical scan based at least partly on the assignment of the data points to classes, and outputting the generated data profile for the medical scan.

FIG. 2

**Description**

Field of the invention

This invention relates to a method and apparatus for generating a data profile for a medical scan.

Background of the invention

In the field of medical imaging, a variety of technologies can be used to investigate biological processes and anatomy. The following examples are types of scan that may be used to provide medical images: X-Ray; Computed Tomography (CT); Ultrasound (US); Magnetic Resonance Imaging (MRI); Single Photon Emission Tomography (SPECT); and Positron Emission Tomography (PET). Each type of scan is referred to as an 'imaging modality'.

Typically, a scan provides a 'dataset'. The dataset comprises digital information about the value of a variable at each of many spatial locations in either a two-dimensional or (more typically) a three-dimensional space. The variable may typically be an intensity measurement. The intensity may be, for example, an indication of the X-Ray attenuation of the tissue at each particular point, or for an MRI scan an indication of a magnetic resonance imaging proton spin-lattice relaxation time.

In the case of a three-dimensional dataset, the element of the scan image located at a particular spatial location is typically referred to as a 'voxel'. A voxel is therefore analogous to a 'pixel' of a conventional 2-dimensional image.

It is to be understood that the term 'image' used herein may refer to either a three-dimensional volumetric image or a two-dimensional planar image, unless otherwise stated or as may be apparent from the context within which the term is used.

Typically, medical scan datasets are viewed as 2-dimensional or 3-dimensional images on a display or as a printed 'hardcopy', with the intensity measurements for the pixels/voxels being represented through the use of different colours (in a colour image) or different shades (e.g. in a grey-scale image). A radiologist or other clinician skilled/experienced in interpreting medical scan images is often able to quickly and accurately decipher and interpret a medical scan image simply upon viewing the image. However, doctors and other medical personnel who are not skilled or experienced in interpreting medical scan images are often required to perform diagnostic and other procedures on patients based on medical scan images. Whilst such medical personnel may be able to easily and quickly decipher basic information from a medical scan image, it is often difficult (if at all possible) for them to fully interpret and comprehend all the information that may be decipherable from a medical scan image for their patient.

Accordingly, there is a need for an improved approach for representing medical scan data that allows the information within medical scan data to be more easily perceived by a user, enabling the user to more easily interpret the medical scan data.

Summary of the invention

According a first aspect of the present invention, there is provided a method of generating a data profile for a medical scan. The method comprises obtaining data point values for a plurality of data points representing spatial locations within the medical scan, determining data point classification parameters defining data point value ranges for a plurality of classes, assigning each data point to a class having a data point value range corresponding to the value for that data point, generating a data profile for the medical scan based at least partly on the assignment of the data points to classes, and outputting the generated data profile for the medical scan.

In this manner, a contribution profile may be generated that allows the distribution of data point values across the ranges defined by the classes to be more easily perceived by a user, enabling the user to more easily interpret the medical scan data.

In accordance with some embodiments, the method may comprise determining a region of interest within the medical scan, assigning each data point within the region of interest to a class having a data point value range corresponding to the value for that data point, and generating a data profile for the region of interest within the medical scan based at least partly on the assignment of the data points within the region of interest to classes.

In accordance with some embodiments, the method may comprise determining a plurality of regions of interest within the medical scan, and for each region of interest, assigning each data point within the region of interest to a class having a data point value range corresponding to the value for that data point, and generating a data profile for the region of interest within the medical scan based at least partly on the assignment of the data points within the region of interest to classes.

In accordance with some embodiments, the, or each, generated data profile may comprise an indication of a number of data points assigned to each class.

In accordance with some embodiments, the method may further comprise computing a proportional contribution value for each class, and generating the data profile for the medical scan based at least partly on the computed proportional contribution values.

In accordance with some embodiments, the, or each, generated data profile may comprise an indication of a proportional contribution for each class.

In accordance with some embodiments, the method may further comprise, for each class within the, or each, data profile, performing at least one evaluation of the proportional contribution value for the class with respect to at least one reference contribution parameter, and generating the data profile for the medical scan based at least partly on the at least one evaluation of the proportional contribution value performed for each class.

In accordance with some embodiments, the at least one reference contribution parameter for each class may define at least one of:

- a reference range;
- a reference proportional contribution value; and
- a reference deviation value.

In accordance with some embodiments, performing the at least one evaluation of the proportional contribution value for each class may comprise at least one of:

- determining whether the proportional contribution value for the class is within a reference range;
- computing a difference between the proportional contribution value for the class and a reference value;
- computing an absolute difference between the proportional contribution value for the class and a reference value;
- computing a proportional difference between the proportional contribution value for the class and a reference value;
- computing a Z-score for the proportional contribution value for the class with respect to a reference mean value and a reference standard deviation value; and
- computing a modified Z-score for the proportional contribution value for the class with respect to a reference median value and a reference median absolute deviation value.

In accordance with some embodiments, the generated data profile may comprise for each class a result of the at least one evaluation of the proportional contribution value for the class.

In accordance with some embodiments, the method may further comprise computing at least one profile assessment measurement with respect to profile assessment data.

In accordance with some embodiments, the at least one profile assessment measurement may comprise at least one of:

- a distance measure between the proportional contribution values for the data profile and the profile assessment data;
- a similarity measure between the proportional contribution values for the data profile and the profile assessment data; and
- a Chi-squared ($X^2$) probability measure between the proportional contribution values for the data profile and the profile assessment data.

In accordance with some embodiments, the generated data profile may comprise the at least one profile assessment measurement.

In accordance with some embodiments, the, or each, generated data profile may be outputted to at least one of:

- a data storage device;
- a printer device; and
- a display device.

According to a second aspect of the present invention, there is provided a non-transitory computer program product having stored therein executable computer program code for generating a data profile for a medical scan, the executable computer program code operable to perform the method of the first aspect of the present invention.

According to a third aspect of the present invention, there is provided a system comprising at least one processing device arranged to obtain data point values for a plurality of data points representing spatial locations within the medical scan, determine data point classification parameters defining data point value ranges for a plurality of classes, assign each data point to a class having a data point value range corresponding to the value for that data point, generate a data profile for the medical scan based at least partly on the assignment of the data points to classes, and output the generated data profile for the medical scan.

Brief description of the drawings

Further details, aspects and embodiments of the invention will be described, by way of example only, with reference to the drawings. In the drawings, like reference numbers are used to identify like or functionally similar elements. Elements in the figures are illustrated for simplicity and clarity and have not necessarily been drawn to scale.

Figure 1 illustrates a simplified overview of an example of a method of generating data profiles for medical scans.
Figure 2 illustrates a simplified flowchart of an example of at least a part of a method for generating a data profile for a medical scan.
Figure 3 illustrates a simplified block diagram of an example of a computer system.
Figure 4 schematically illustrates a simplified example of the execution of computer program code for generating a data profile for a medical scan.
Figure 5 illustrates a simplified flowchart of an example of a method of performing a contribution assessment.
Figure 6 illustrates a simplified flowchart of an example of a method of performing a profile assessment.
Figure 7 illustrates an example of a data profile for a medical scan.

Detailed description of the preferred embodiments

The present invention will now be described with reference to the accompanying drawings. However, it will be appreciated that the present invention is not limited to the specific examples herein described and as illustrated in the accompanying drawings, and various modifications and alterations may be made without departing from the inventive concept. Furthermore, because the illustrated embodiments of the present invention may for the most part, be implemented using electronic components and circuits known to those skilled in the art, details will not be explained to any greater extent than that considered necessary as illustrated below, for the understanding and appreciation of the underlying concepts of the present invention and in order not to obfuscate or distract from the teachings of the present invention.

In accordance with some examples of the present invention, there is provided a method and apparatus for generating a data profile for a medical scan based on the assignment of data points (e.g. pixels/voxels) within the medical scan into classes. Each data point is assigned to a class having a data point value range corresponding to the value for that data point. In this manner, a contribution profile may be generated that allows the distribution of data point values across the ranges defined by the classes to be more easily perceived by a user, enabling the user to more easily interpret the medical scan data.

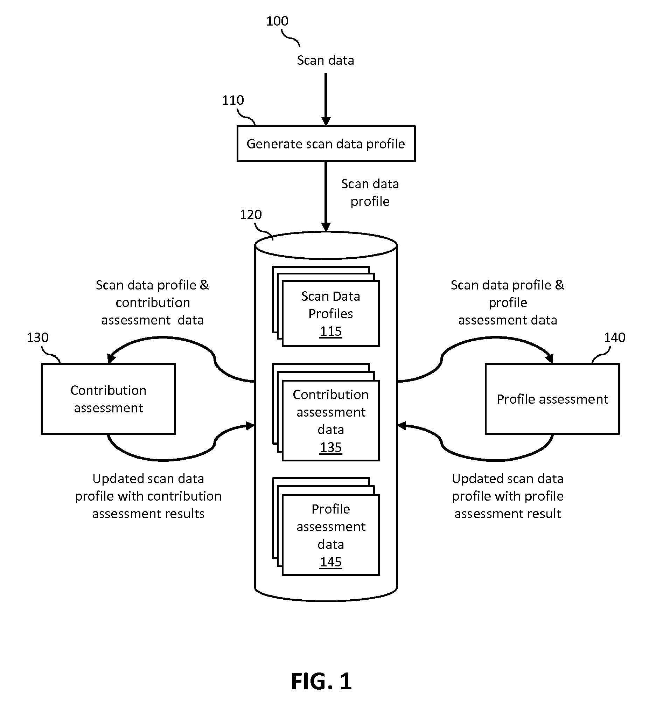

Referring now to Figure 1, there is illustrated a simplified overview of an example of a method of generating data profiles for medical scans in accordance with examples of the present invention. Scan data 100 comprising data point values for a plurality of data points representing spatial locations within a medical scan is loaded, or otherwise obtained. The data point values may be in the form of intensity measurements corresponding to the imaging modality used to obtain the scan data, and the scan data may relate to 2-dimensional (planar) or 3-dimensional (volumetric) spatial locations. For example, data point values for a magnetic resonance imaging (MRI) scan may comprise measurement values indicating magnetic resonance imaging proton spin-lattice relaxation times at the respective spatial locations. Alternatively, for an X-ray scan the scan data values may comprise indications of the X-Ray attenuation of the tissue at the respective spatial locations. A data profile for the scan data 100 is then generated, as indicated generally at 110, and in the illustrated example stored within a data storage device 120.

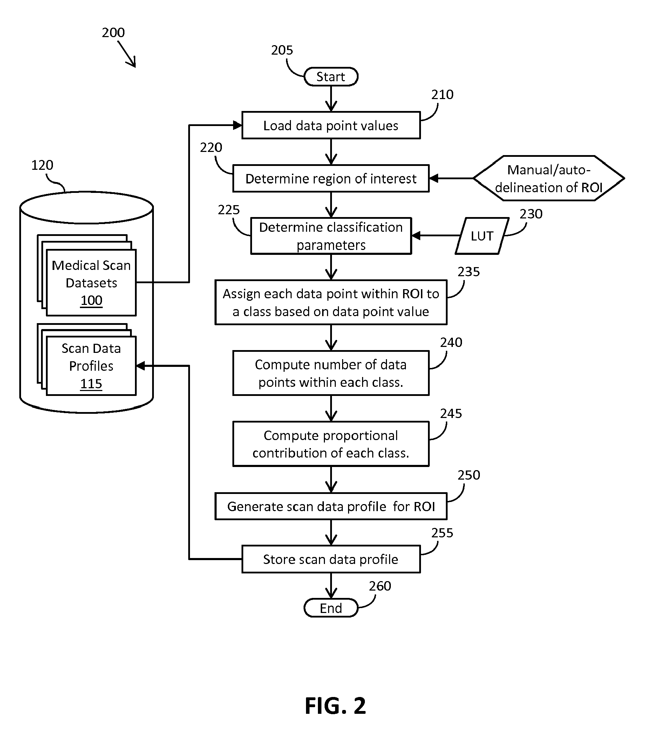

Figure 2 illustrates a simplified flowchart 200 of an example of at least a part of a method for generating a data profile for a medical scan, such as may be performed at 110 in Figure 1. The method starts at 205, and moves on to 210 where, in the example illustrated in Figure 2, scan data 100 in the form of data point values 215 for data points representing spatial locations within the medical scan are loaded from the data storage device 120. In the illustrated example, a region of interest within the medical scan is determined, at 220. The region of interest may be manually defined by a user or may be automatically defined, for example by way of an auto-contouring program arranged to generate contours that delineate structures (e.g. organs etc.) within a medical scan. It is contemplated that a region of interest may define a part of the medical scan or may consist of the medical scan as a whole.

Data point classification parameters defining data point value ranges for a plurality of classes are then determined, at 220. In the example illustrated in Figure 2, the data point classification parameters are retrieved from a lookup table 230. For example, the lookup table 230 may contain data point classification parameters relating to different medical scan modalities etc. and the data point classification parameters for the relevant medical scan modality for the medical scan for which a data profile is being generated are retrieved at 225.

Table 1 below illustrates one example of data point classification parameters for T1 relaxation times from an MRI scan.

Table 1

| Relaxation time | Biological Significance | Class |
|---|---|---|
| <650ms | Non-liver tissue, e.g. fat | Sub-LIF |
| 650-800ms | Healthy liver parenchyma | LIF 1 |
| 800-875ms | Mild inflammation and fibrosis | LIF 2 |
| 875-950ms | Moderate inflammation & fibrosis and/or small blood vessels | LIF 3 |
| 950-1200ms | Severe inflammation & fibrosis and/or substantial blood vessels | LIF 4 |
| >1200ms | Bile | Super-LIF |

In the first (left-hand) column, relaxation times have been divided into ranges that have been found to correspond to particular biological implications, described in the second column. Each of these relaxation time ranges is then associated with a particular class. In the example of Table 1, six classes are defined: four LIF (liver inflammation and fibrosis) classes; a sub-LIF class and a super-LIF class. Thus, the classification parameters derived at 225 in Figure 2 may comprise a class identifier (e.g. a simple index value or a name) and parameters defining each data point value range (e.g. the relaxation time ranges in the example of Table 1).

Having determined the data point classification parameters defining class data point value ranges, each data point within the defined region of interest is assigned to the relevant class having a range corresponding to the value for that data point. Advantageously, by assigning the data points in to classes in this manner, a more informative interpretation of the scan data may begin to be made based on, for example, a proportional distribution of the data points amongst the different classes. Accordingly, in the example illustrated in Figure 2, having assigned all of the data points within the defined region of interest to their appropriate classes, the method moves on to 240 where the number of data points assigned to each class is computed. A proportional contribution for each class is then computed at 245. A data profile 115 for the defined region of interest within the medical scan is then generated at 250 comprising, for example, the number of data points assigned to each class, the proportional contribution for each class, etc. The generated data profile 115 is then stored in the data storage device 120, at 255. The generated profile may additionally/alternatively be output to, say, a display device where the data profile is displayed or a printer device where a hard copy (e.g. paper copy) of the data profile is printed. The method of Figure 2 then ends, at 260.

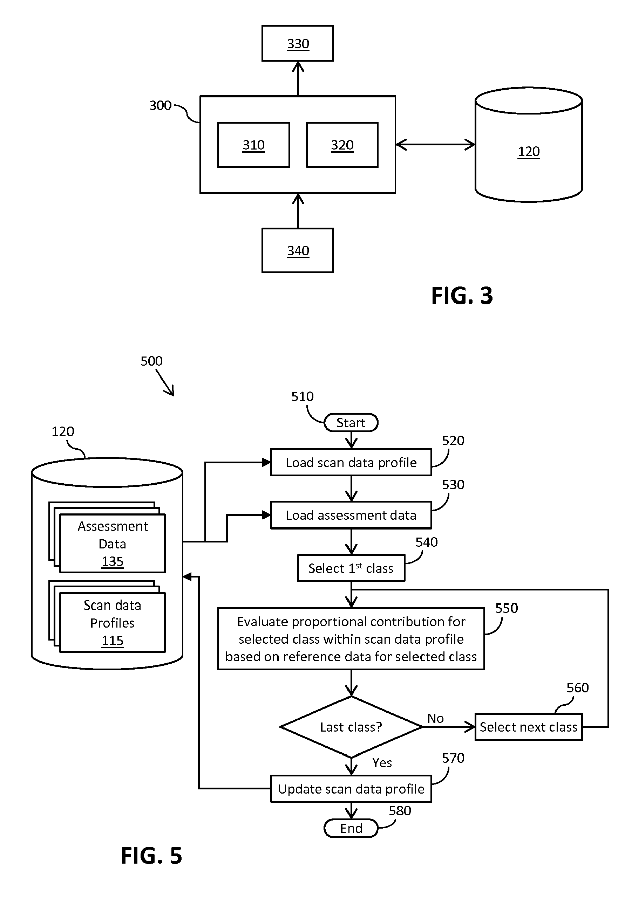

Figure 3 illustrates a simplified block diagram of an example of a computer system 300 that may be adapted in accordance with examples of the present invention. The system 300 comprises one or more processing devices 310 arranged to execute computer program code. The system 300 further comprises one or more memory elements 320. The memory element(s) 320 may consist of one or more non-transitory computer program products such as, for example, a hard disk, an optical storage device such as a CD-ROM device, a magnetic storage device, a Read Only Memory, ROM, a Programmable Read Only Memory, PROM, an Erasable Programmable Read Only Memory, EPROM, an Electrically Erasable Programmable Read Only Memory, EEPROM, and a Flash memory, etc. The memory element 320 may additionally/alternatively comprise one or more volatile memory elements such as, for example, Random Access Memory (RAM), cache memory, etc.

For simplicity and ease of understanding, a single processing device 310 and a single memory element 320 will hereinafter be referred to. However, it will be appreciated that such references to a single processing device 310 or a single memory element 320 are intended to encompass multiple processing devices 310 and multiple memory elements 320 respectively.

The memory element 320 may have stored therein executable computer program code to be executed by the processing device 310. The memory element 320 may further have stored therein data to be accessed and/or processed by the processing device 310 when executing computer program code.

The system 300 further comprises one or more output devices, indicated generally at 330. Such output devices may comprise, by way of example, a display device, a printer device, a network interface device, etc. The system 300 further comprises one or more user input devices, indicated generally at 340. Such input devices may include, by way of example, a keyboard, a keypad, a mouse, a touchscreen, etc.

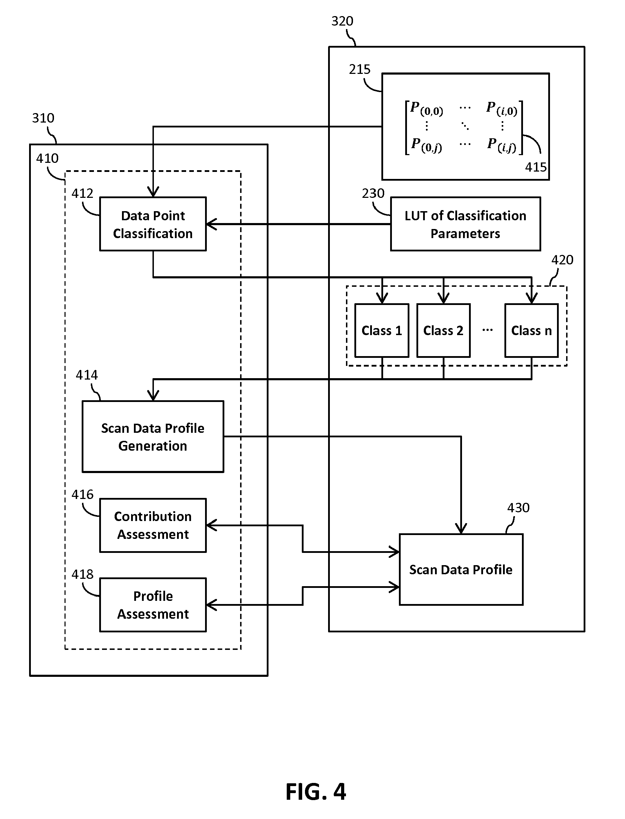

In accordance with some examples of the present invention, the processing device 310 is arranged to execute computer program code stored within the memory element 320 for generating data profiles for medical scans. Figure 4 schematically illustrates a simplified example of the execution of such computer program code 410 within the computer system 300 by the processing device 310. In the example illustrated in Figure 4, the computer program code 410 comprises a data point classification component 412 arranged to read data point values 215 for a medical scan from the memory element 320, and assign each of the respective data points to an appropriate class, for example as described above in relation to step 235 of Figure 2.

In some examples, the area of memory 320 illustrated in Figure 4 comprises memory directly accessible by the processing device 310, such as RAM or cache memory or a combination of both. The data point values 215 may previously have been loaded from a local or remote data storage device, such as the data storage device 120 illustrated in Figure 1, into memory 320. Alternatively, the data point classification component 412 may be arranged to load the data point values 215 into memory 320. In the example illustrated in Figure 4, the data point values comprise values for a plurality of data points representing 2-dimensional spatial locations within a planar medical scan. In alternative examples, the data point values may comprise values for a plurality of data points representing 3-dimensional spatial locations within a volumetric medical scan.

In the example illustrated in Figure 4, the data point classification component 412 is arranged to read only those data point values 415 representing spatial locations within a predefined region of interest of the medical scan. The data point classification component 412 reads each of the data point values 415 for the region of interest, and assigns the respective data point to one of a plurality of classes 420 based on data point classification parameters held within the lookup table 230. The data point classification component 412 may assign each data point to a class by writing a data point identifier, such as an (x,y) coordinate value for 2-dimensional medical scan data, for the respective data point to a data structure in memory 320 for the respective class. Additionally/alternatively, the data point classification component 412 may be arranged to increment a counter value for the respective class; the counter values for the classes 420 being stored within memory 320, or alternatively maintained within one or more registers (not shown) of the processing device 310. Such counter values maintained within registers of the processing device 310 may subsequently be written to memory 320 once the data point classification component 412 has finished assigning the data points to classes.

The computer program code 410 further comprises a scan data profile generation component 414 arranged to generate a data profile for the medical scan based on the assignment of the data points 415 to respective classes 420. For example, if the data point classification component 412 was not arranged to increment a counter value for the respective class for each assignment of a data point to a class, the scan data profile generation component 414 may be arranged to compute the number of data points within each class 420. The scan data profile generation component 414 may further be arranged to compute proportional contributions for each class. For example, the scan data profile generation component 414 may compute the total number of data points assigned to all classes, and then for each class divide the number of data points assigned to that class by the total number of assigned data points to compute the proportion of data points assigned to that class ('the proportional contribution'). The scan data profile generation component 414 may then generate a data profile 430 for the medical scan comprising the computed values. For example, the data profile 430 may include, for each class, an indication of the number of data points assigned to that class and the proportional contribution of data points assigned to that class (e.g. a percentage of the total number of assigned data points assigned to that class).

Referring back to Figure 1, a contribution assessment may be performed on the assignment of data points to groups for a data profile, as indicated generally at 130. For the example illustrated in Figure 1, performing such a contribution assessment 130 comprises retrieving a data profile 115 previously generated at 110 from the data storage device 120, along with contribution assessment data 135, and performing an evaluation of the proportional contribution for each class based on the contribution assessment data 135. In this manner, the contribution assessment 130 individually assesses the proportional contributions of each class of a data profile 115. The data profile 115 is then updated with the results of the contribution assessment, and written back to the data storage device.

Figure 5 illustrates a simplified flowchart 500 of an example of a method of performing a contribution assessment, such as may be performed at 130 in Figure 1. The method starts at 510, and moves on to 520 where a data profile 115 is loaded from the data storage device 120. Contribution assessment data 135 is then loaded (or otherwise obtained) at 530 comprising reference contribution parameters for each class of the data profile 115.

The reference contribution parameters for each class may define, for example, one or more of:

- a reference range;
- a reference (e.g. mean or median) proportional contribution value;
- a reference deviation value (e.g. a standard deviation value or median absolute deviation value);
- etc.

It is contemplated that multiple sets of contribution assessment data 135 may be stored within the data storage device 120. For example, different sets of contribution assessment data 135 may be stored corresponding to different scanning modalities and for different parts of the human anatomy. Furthermore, different contribution assessment data sets 135 may be available representing different segments of the population (e.g. representing different age ranges, genders, ethnicity, etc.) and/or representative of different conditions etc. For example, different MRI related contribution assessment data 135 sets may be available for the liver representative of different conditions such as portal hypertension, cirrhosis, fibrosis, inflammation, potentially subdivided into contributing etiologies, e.g. autoimmune hepatitis, primary biliary cirrhosis, primary sclerosing cholangitis, viral hepatitis, chronic hepatitis, drug-induced hepatitis, radiation-induced liver disease, haemochromatosis, thallassaemia, alcoholic hepatitis, alcoholic liver cirrhosis, portal hypertension, vascular liver disease, idiopathic hepatic fibrosis, sarcoidosis, hepatic cysts, and hemangiomas. viral and autoimmune hepatitis, obesity, alcoholism. Contribution assessment data 135 sets may also be generated for specific studies, for example treated or untreated individuals in a clinical trial, and for individuals who are monitored repeatedly on a longitudinal basis. A specific contribution data set may be selected manually by a user, or autonomously based on information entered by a user or otherwise obtained such as from a patient's medical record etc.

Having loaded (or otherwise obtained) assessment data 135, a first class for the data profile 115 is then selected at 540, and an evaluation of the proportional contribution for the selected class is performed with respect to the reference contribution parameters within the contribution assessment data 135 for the selected class, at 550. For example, if the reference contribution parameters define a reference range, the proportional contribution for the selected class may be evaluated by determining whether the proportional contribution for the selected class is within, above or below the reference range. Additionally/alternatively, if the reference contribution parameters define a reference proportional contribution value and/or a reference deviation value, the proportional contribution for the selected class may be evaluated by computing one or more of a difference, an absolute difference, a proportional difference, the Z-score or modified Z-score, or other deviation metric. For example, a Z-score may be given by:

$$Z(x_i, \Theta) = \frac{x_i - \mu_\Theta}{\sigma_\Theta} \qquad \text{Equation 1}$$

where $\Theta$ represents the reference statistics for the selected class defined within the reference contribution parameters and $x_i$ the proportional contribution value for the selected class within the data profile 115. A modified Z-score may be given by:

$$MZ(x_i, \Theta) = \frac{x_i - |median(\Theta)|}{mad(\Theta)} \qquad \text{Equation 2}$$

where *MAD* is the median absolute deviation, given by:

$$MAD(\Theta) = median\left(\left| \Theta_i - median_j(\Theta_j) \right|\right) \qquad \text{Equation 3}$$

Having performed the evaluation of the proportional contribution value for the selected class, if the selected class is not the last class for the data profile 115, the next class is selected at 560, and the method loops back to 550 where the proportional contribution value for the newly selected class is evaluated. Once the proportional contribution values for every class have been evaluated, the method moves on to 570 where the scan data profile 115 stored within the data storage device 120 is updated to include the results of the evaluations of the contribution assessment values for each class. The method then ends at 580.

In this manner, the data profile 115 may be updated to include evaluation information for the proportional contribution of data points within each individual class, allowing the distribution of data point values across the classes to be more easily interpreted by a user.

Referring back to Figure 1, a profile assessment may be performed on the assignment of data points to groups for a data profile 115, as indicated generally at 140. For the example illustrated in Figure 1, performing such a profile assessment 140 comprises retrieving a data profile 115 previously generated at 110 from the data storage device 120, along with profile assessment data 145, and performing an evaluation of the data profile 115 to provide a compound or unified single measure of a data profile's similarity or closeness to, for example, a particular reference population. The data profile 115 is then updated with the results of the contribution assessment, and written back to the data storage device 120.

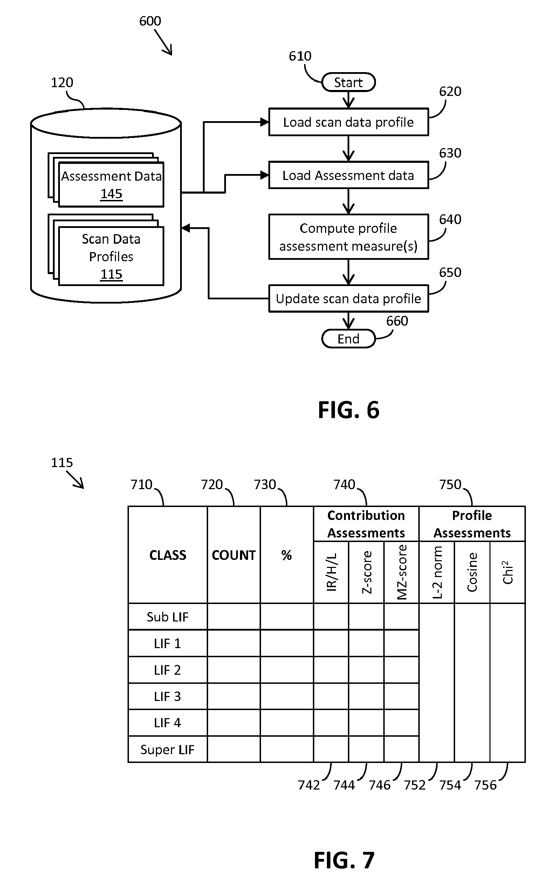

Figure 6 illustrates a simplified flowchart 600 of an example of a method of performing a profile assessment, such as may be performed at 140 in Figure 1. The method starts at 610, and moves on to 620 where a data profile 115 is loaded from the data storage device 120. Profile assessment data 145 is then loaded (or otherwise obtained) at 630 comprising reference profile parameters.

The reference profile parameters may define, for example, a reference proportional contribution value for each class representing a profile corresponding to a reference population.

It is contemplated that multiple sets of profile assessment data 145 may be stored within the data storage device 120. For example, different sets of profile assessment data 145 may be stored corresponding to different scanning modalities and for different parts of the human anatomy. Furthermore, different profile assessment data sets 145 may be available representing different segments of the population (e.g. representing different age ranges, genders, ethnicity, etc.) and/or representative of different conditions etc. For example, different MRI related profile assessment data 145 sets may be available for the liver representative of different conditions such as portal hypertension, cirrhosis, fibrosis, inflammation, potentially subdivided into contributing etiologies, e.g. autoimmune hepatitis, primary biliary cirrhosis, primary sclerosing cholangitis, viral hepatitis, chronic hepatitis, drug-induced hepatitis, radiation-induced liver disease, haemochromatosis, thallassaemia, alcoholic hepatitis, alcoholic liver cirrhosis, portal hypertension, vascular liver disease, idiopathic hepatic fibrosis, sarcoidosis, hepatic cysts, and hemangiomas. viral and autoimmune hepatitis, obesity, alcoholism. Profile assessment data 145 sets may also be generated for specific studies, for example treated or untreated individuals in a clinical trial, and for individuals who are monitored repeatedly on a longitudinal basis. A specific contribution data set may be selected manually by a user, or autonomously based on information entered by a user or otherwise obtained such as from a patient's medical record etc.

In some examples of the present invention, it is contemplated that there may be a degree of overlap between the contribution assessment data 135 and the profile assessment data 145, whereby some data may be used for performing both the contribution assessment 130 for a data profile 115 and the profile assessment 140 of the data profile.

Having loaded (or otherwise obtained) the assessment data 145, a profile assessment measure is computed for the data profile 115 based on the assessment data 145. Such a profile assessment measure may include, for example, a distance measure or similarity measure between the proportional contributions of the classes within the data profile 115 and the reference proportional contribution values defined in the profile assessment data 145. Examples of such distance measures include, by way of example only:

- an L-2 norm measurement (the square root of the sum of squares of the differences between the data profile (p(x)) class contribution values and corresponding reference profile (q(x)) class contribution values, with d number of classes or features);

$$\sqrt{\sum_{i=1}^{d} (p_i(x) - q_i(x))^2}$$

- an L-1 norm measurement (the sum of the differences - also known as the Manhattan distance);

$$\sum_{i=1}^{d} |p_i(x) - q_i(x)|$$

- an L-infinity norm measurement (the maximum of differences in any of the classes);

$$\max_i |p_i(x) - q_i(x)|$$

- a Minkowski distance (the $m^{th}$ root of the sum of the differences raised to the $m^{th}$ power);

$$\sqrt[m]{\sum_{i=1}^{d} |p_i(x) - q_i(x)|^m}$$

- a Laplacian distance given by

$$\frac{<p(x).q(x)>}{\sqrt{<p(x).p(x)><q(x).q(x)>}}$$

- a Mahalanobis distance (a measure of the distance of the profile from a population of profiles by measuring how many standard deviations away the sample is from the population, taking into account the correlations within the classes given by covariance matrix S);

$$\sqrt{\sum_{i=1}^{d}(p_i(x) - \mu_{q_i(x)})(p_i(x) - \mu_{q_i(x)})S^{-1}}$$

- etc.

Examples of similarity measures include, by way of example only:

- a Bhattacharyya coefficient (a measure of the amount of overlap between the data profile class contribution values and corresponding reference contribution values);

$$\sum_{i=1}^{d}\sqrt{p_i(x)q_i(x)}$$

- Jaccard Index (defined as the size of intersection of sample sets divided by the size of union of the sample sets);

$$\frac{|\cap(p(x),q(x)|}{|\cup(p(x),q(x)|}$$

- the cosine similarity (a measure of the cosine of the angle between the data profile and reference contribution vectors);

$$\cos\theta = \frac{<p(x).q(x)>}{\|p(x)\|\|q(x)\|}$$

- the Pearson Correlation measure (a measure of the linear correlation (dependence) between the data profile and reference contribution vectors)

$$\frac{\sum_1^d(p_i(x) - \overline{p(x)})(q_i(x) - \overline{q(x)})}{\sqrt{\sum(p_i(x) - \overline{p(x)})^2}\sqrt{\sum(q_i(x) - \overline{q(x)})^2}}$$

- information theoretic similarity measures including but not limited to:

  ∘ Joint entropy
  ∘ Conditional entropy
  ∘ Mutual information

- etc.

A further alternative profile assessment measure may include a Chi-squared ($X^2$) probability.
Having computed the profile assessment measure(s), the scan data profile 115 stored within the data storage device 120 is updated to include the profile assessment measure(s) at 650. The method then ends at 660.
In this manner, the data profile 115 may be updated to comprise a measure of how similar and/or different a data profile for a medical scan is to a profile of a particular reference population (e.g. a healthy reference population, a reference population for a particular disease, etc.). Such a measure of how similar and/or different a data profile for a medical scan is to a particular reference population enables a user to more easily assess a patient's condition relative to the corresponding reference population.
Figure 7 illustrates an example of a data profile 115. In the example illustrated in Figure 7 the data profile 115 is generated based on the assignment of data points within a medical scan into six classes 710: four LIF (liver inflammation and fibrosis) classes; a sub-LIF class and a super-LIF class, such as as defined in Table 1 above. In the illustrated example, the data profile 115 contains an indication 720 of the number of data points assigned to each class. The data profile 115 further contains an indication 730 of the proportional contribution of each class (e.g. the percentage of the total number of data points that have been assigned to each class).

The data profile 115 illustrated in Figure 7 further includes one or more contribution assessment results 740 for each class. In particular, the data profile 115 illustrated in Figure 7 comprises, for each class:

- an indication 742 of whether the proportional contribution is in range (IR), higher than (H) or lower than (L) a reference range;
- a Z-score 744; and
- a modified z-score 746.

The data profile 115 illustrated in Figure 7 further includes one or more profile assessment measures 750. In particular, the data profile 115 illustrated in Figure 7 comprises:

- a L-2 norm measure 752;
- a cosine similarity measure 754; and
- a Chi-squared probability value 756.

The data profile 115 may be stored, for example within the data storage element 120, in any suitable format. For example, the data profile 115 may be stored as a MATLAB (matrix laboratory) data structure (e.g. within a .mat file). In other examples, the data profile 115 may be stored within the DICOM (Digital Imaging and Communications in Medicine) file of the medical scan to which it relates, for example within private tags of the DICOM file.

A data profile generated in accordance with examples of the present invention, such as the data profile illustrated in Figure 7, provides a user with statistical information relating to a medical scan, such as:

- the number of data points assigned to each of a plurality of classes;
- an indication of the proportional contribution of each class;
- one or more contribution assessment results for each class; and
- one or more profile assessment measures.

Advantageously, such statistical information enables a user such as a doctor or other medical personnel not skilled or experienced in interpreting medical scan images to more easily interpret and comprehend information relating to a medical scan that they may otherwise find difficult to decipher from the medical scan data itself.

It is contemplated that such data profiles may be viewed or otherwise accessed by a user in isolation, providing the user with the statistical information contained therein. In some examples, the information contained within a data profile may be converted into one or more visualisations of the data. For example, the information contained within a data profile may converted into a modified pie chart, whereby each class is represented by a segment representing the proportional contribution of the class. In some examples, the radius of each segment is dependent on, say, a contribution assessment result for the corresponding class. For example, if the data profile includes contribution assessment results indicating whether the proportional contribution for each class is in range (IR), higher than (H) or lower than (L) a reference range, each segment corresponding to a class for which the proportional contribution is in range may have a default radius. By contrast, each segment corresponding to a class for which the proportional contribution is higher than the reference range may have an increased radius (relative to the default radius), whilst each segment corresponding to a class for which the proportional contribution is lower than the reference range may have a reduced radius (relative to the default radius). In this manner, the information contained within a data profile may be used to generate a visualization of the scan data that enables a user to quickly and easily identify characteristics of the scan data.

Alternatively, a data profile may be presented to a user along with one or more reference profiles, enabling the user to compare the data profile for a patient's medical scan to the one or more reference profiles. In this manner, the user is able to more easily assess the statistical information contained within the data profile.

For simplicity and ease of understanding, the contribution assessment 130 and profile assessment 140 have been illustrated and hereinbefore described as methods performed separately with respect to each other, and with respect to the generation of the data profile 115. However, it is contemplated that the contribution assessment 130 and/or the profile assessment may equally form an integral part of the method of generating the data profile 115. For example, the steps of the methods illustrated in Figures 5 and/or 7 may be integrated into the method illustrated in Figure 2, such that data profile 115 may be initially generated to include contribution assessment results and/or profile assessment measure(s), as opposed to subsequently being updated to include the contribution assessment results and/or profile assessment measure(s) as illustrated in Figure 1.

Furthermore, it is contemplated that the contribution assessment 130 and/or profile assessment may be repeatedly performed, for example using different assessment data sets 135, 145, or as assessment data 135, 145 is updated over time.

In the example illustrated in Figure 2, a scan data profile is generated in relation to a region of interest defined within the medical scan. It is contemplated that, where multiple regions of interest are present within a medical scan, a scan data profile may be generated for each region of interest.

The present invention has been hereinbefore described primarily with reference to an MRI scan, with the scan data values therefore comprising indications of magnetic resonance imaging proton spin-lattice relaxation times. However, it will be understood that the present invention is not limited to being implemented in relation to MRI scans, and may equally be applied to scan data obtained by way of any medical imaging modality and in particular to any medical scan data comprising quantitative data values.

As herein before described, the invention may also be implemented in a computer program for running on a computer system, at least including code portions for performing steps of a method according to the invention when run on a programmable apparatus, such as a computer system or enabling a programmable apparatus to perform functions of a device or system according to the invention.

A computer program is a list of instructions such as a particular application program and/or an operating system. The computer program may for instance include one or more of: a subroutine, a function, a procedure, an object method, an object implementation, an executable application, an applet, a servlet, a source code, an object code, a shared library/dynamic load library and/or other sequence of instructions designed for execution on a computer system.

The computer program may be stored internally on a tangible and non-transitory computer readable storage medium or transmitted to the computer system via a computer readable transmission medium. All or some of the computer program may be provided on computer readable media permanently, removably or remotely coupled to an information processing system. The tangible and non-transitory computer readable media may include, for example and without limitation, any number of the following: magnetic storage media including disk and tape storage media; optical storage media such as compact disk media (e.g., CD-ROM, CD-R, etc.) and digital video disk storage media; non-volatile memory storage media including semiconductor-based memory units such as FLASH memory, EEPROM, EPROM, ROM; ferromagnetic digital memories; MRAM; volatile storage media including registers, buffers or caches, main memory, RAM, etc.

A computer process typically includes an executing (running) program or portion of a program, current program values and state information, and the resources used by the operating system to manage the execution of the process. An operating system (OS) is the software that manages the sharing of the resources of a computer and provides programmers with an interface used to access those resources. An operating system processes system data and user input, and responds by allocating and managing tasks and internal system resources as a service to users and programs of the system.

The computer system may for instance include at least one processing unit, associated memory and a number of input/output (I/O) devices. When executing the computer program, the computer system processes information according to the computer program and produces resultant output information via I/O devices.

In the foregoing specification, the invention has been described with reference to specific examples of embodiments of the invention. It will, however, be evident that various modifications and changes may be made therein without departing from the scope of the invention as set forth in the appended claims and that the claims are not limited to the specific examples described above.

Those skilled in the art will recognize that the boundaries between logic blocks are merely illustrative and that alternative embodiments may merge logic blocks or circuit elements or impose an alternate decomposition of functionality upon various logic blocks or circuit elements. Thus, it is to be understood that the architectures depicted herein are merely exemplary, and that in fact many other architectures can be implemented which achieve the same functionality.

Any arrangement of components to achieve the same functionality is effectively 'associated' such that the desired functionality is achieved. Hence, any two components herein combined to achieve a particular functionality can be seen as 'associated with' each other such that the desired functionality is achieved, irrespective of architectures or intermediary components. Likewise, any two components so associated can also be viewed as being 'operably connected,' or 'operably coupled,' to each other to achieve the desired functionality.

Furthermore, those skilled in the art will recognize that boundaries between the above described operations merely illustrative. The multiple operations may be combined into a single operation, a single operation may be distributed in additional operations and operations may be executed at least partially overlapping in time. Moreover, alternative embodiments may include multiple instances of a particular operation, and the order of operations may be altered in various other embodiments.

However, other modifications, variations and alternatives are also possible. The specifications and drawings are, accordingly, to be regarded in an illustrative rather than in a restrictive sense.

In the claims, any reference signs placed between parentheses shall not be construed as limiting the claim. The word 'comprising' does not exclude the presence of other elements or steps then those listed in a claim. Furthermore, the terms 'a' or 'an,' as used herein, are defined as one or more than one. Also, the use of introductory phrases such as 'at least one' and 'one or more' in the claims should not be construed to imply that the introduction of another claim element by the indefinite articles 'a' or 'an' limits any particular claim containing such introduced claim element to inventions containing only one such element, even when the same claim includes the introductory phrases 'one or more' or 'at least one' and indefinite articles such as 'a' or 'an.' The same holds true for the use of definite articles. Unless stated otherwise, terms such as 'first' and 'second' are used to arbitrarily distinguish between the elements such terms describe. Thus, these terms are not necessarily intended to indicate temporal or other prioritization of such elements. The mere fact that certain measures are recited in mutually different claims does not indicate that a combination of these measures cannot be used to advantage.

**Claims**

1. A method of generating a data profile for a medical scan, the method comprising;
obtaining data point values for a plurality of data points representing spatial locations within the medical scan;
determining data point classification parameters defining data point value ranges for a plurality of classes;
assigning each data point to a class having a data point value range corresponding to the value for that data point;
generating a data profile for the medical scan based at least partly on the assignment of the data points to classes; and
outputting the generated data profile for the medical scan.

2. The method of Claim 1, wherein the method comprises:

determining a region of interest within the medical scan;
assigning each data point within the region of interest to a class having a data point value range corresponding to the value for that data point; and
generating a data profile for the region of interest within the medical scan based at least partly on the assignment of the data points within the region of interest to classes.

3. The method of Claim 2, wherein the method comprises determining a plurality of regions of interest within the medical scan, and for each region of interest:

assigning each data point within the region of interest to a class having a data point value range corresponding to the value for that data point; and
generating a data profile for the region of interest within the medical scan based at least partly on the assignment of the data points within the region of interest to classes.

4. The method of any one of the preceding Claims, wherein the method further comprises computing a proportional contribution value for each class, and generating the data profile for the medical scan based at least partly on the computed proportional contribution values.

5. The method of Claim 4, wherein the method further comprises, for each class within the, or each, data profile:

performing at least one evaluation of the proportional contribution value for the class with respect to at least one reference contribution parameter; and
generating the data profile for the medical scan based at least partly on the at least one evaluation of the proportional contribution value performed for each class.

6. The method of Claim 5, wherein the at least one reference contribution parameter for each class defines at least one of:

a reference range;
a reference proportional contribution value; and
a reference deviation value.

7. The method of Claim 5 or Claim 6, wherein performing the at least one evaluation of the proportional contribution value for each class comprises at least one of:

determining whether the proportional contribution value for the class is within a reference range;

computing a difference between the proportional contribution value for the class and a reference value;

computing an absolute difference between the proportional contribution value for the class and a reference value;

computing a proportional difference between the proportional contribution value for the class and a reference value;

computing a Z-score for the proportional contribution value for the class with respect to a reference mean value and a reference standard deviation value; and

computing a modified Z-score for the proportional contribution value for the class with respect to a reference median value and a reference median absolute deviation value.

8. The method of any one of Claims 5 to 7, wherein the generated data profile comprises for each class a result of the at least one evaluation of the proportional contribution value for the class.

9. The method of any one of Claims 4 to 8, wherein the method further comprises computing at least one profile assessment measurement with respect to profile assessment data.

10. The method of Claim 9, wherein the at least one profile assessment measurement comprises at least one of:

a distance measure between the proportional contribution values for the data profile and the profile assessment data;

a similarity measure between the proportional contribution values for the data profile and the profile assessment data; and

a Chi-squared ($X^2$) probability measure between the proportional contribution values for the data profile and the profile assessment data.

11. The method of any one of the preceding Claims, wherein the, or each, generated data profile is outputted to at least one of:

a data storage device;
a printer device; and
a display device.

12. The method of any one of the preceding Claims, wherein the generated data profile comprises at least one of:

an indication of a number of data points assigned to each class;
an indication of a proportional contribution for each class; and
at least one profile assessment measurement.

13. A non-transitory computer program product having stored therein executable computer program code for generating a data profile for a medical scan, the executable computer program code operable to perform the method of any one of the preceding Claims.

14. The non-transitory computer program product of Claim 15, wherein the non-transitory computer program product comprises one of a hard disk, an optical storage device, a magnetic storage device, a Read Only Memory, a Programmable Read Only Memory, an Erasable Programmable Read Only Memory, an Electrically Erasable Programmable Read Only Memory, and a Flash memory.

15. A system comprising at least one processing device arranged to:

obtain data point values for a plurality of data points representing spatial locations within the medical scan;
determine data point classification parameters defining data point value ranges for a plurality of classes;
assign each data point to a class having a data point value range corresponding to the value for that data point;
generate a data profile for the medical scan based at least partly on the assignment of the data points to classes; and
output the generated data profile for the medical scan.

FIG. 1

200

205

Start

210

Load data point values

220

Determine region of interest

Manual/auto-
delineation of ROI

230

225

Determine classification
parameters

LUT

120

235

Assign each data point within ROI to
a class based on data point value

Medical Scan
Datasets
100

240

Compute number of data
points within each class.

245

Scan Data
Profiles
115

Compute proportional
contribution of each class.

250

Generate scan data profile for ROI

255

Store scan data profile

260

End

**FIG. 2**

330

300

310   320

120

FIG. 3

500

510

Start

120

Assessment
Data
135

Scan data
Profiles
115

520

Load scan data profile

530

Load assessment data

540

Select 1$^{st}$ class

550

Evaluate proportional contribution for
selected class within scan data profile
based on reference data for selected class

Last class?   No   Select next class   560

Yes

570

Update scan data profile

580

End

FIG. 5

320

215

$$\begin{bmatrix} P_{(0,0)} & \cdots & P_{(i,0)} \\ \vdots & \ddots & \vdots \\ P_{(0,j)} & \cdots & P_{(i,j)} \end{bmatrix}$$

415

310

410

412

Data Point
Classification

230

LUT of Classification
Parameters

420

Class 1 | Class 2 | ··· | Class n

414

Scan Data Profile
Generation

416

Contribution
Assessment

430

Scan Data Profile

418

Profile
Assessment

## FIG. 4

**FIG. 6**

| CLASS | COUNT | % | Contribution Assessments | | | Profile Assessments | | |
|---|---|---|---|---|---|---|---|---|
| | | | IR/H/L | Z-score | MZ-score | L-2 norm | Cosine | Chi² |
| Sub LIF | | | | | | | | |
| LIF 1 | | | | | | | | |
| LIF 2 | | | | | | | | |
| LIF 3 | | | | | | | | |
| LIF 4 | | | | | | | | |
| Super LIF | | | | | | | | |

**FIG. 7**

Europäisches
Patentamt

European
Patent Office

Office européen
des brevets

# EUROPEAN SEARCH REPORT

Application Number

EP 16 18 5927

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/156280 A1 (KADIR TIMOR [GB]) 20 June 2013 (2013-06-20) * abstract * * figures 1,5-7 * * paragraphs [0045], [0053] - [0063], [0084], [0085], [0094] - [0104], [0107], [0109] * * claims 1-4,7 * | 1-15 | INV. G06T7/00 |
| X | VINCENZA CALVARUSO ET AL: "Computer-assisted image analysis of liver collagen: Relationship to Ishak scoring and hepatic venous pressure gradient", HEPATOLOGY, vol. 49, no. 4, 19 November 2008 (2008-11-19), pages 1236-1244, XP055325291, ISSN: 0270-9139, DOI: 10.1002/hep.22745 * abstract * * page 1238 - page 1240 * * figures 1,2 * | 1-15 | |
| | | | TECHNICAL FIELDS SEARCHED (IPC) |
| A | US 2013/317341 A1 (SPIES LOTHAR [DE]) 28 November 2013 (2013-11-28) * the whole document * | 1-15 | G06T |

The present search report has been drawn up for all claims

| Place of search | Date of completion of the search | Examiner |
|---|---|---|
| Munich | 19 December 2016 | Eveno, Nicolas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 18 5927

This annex lists the patent family members relating to the patent documents cited in the above-mentioned European search report.
The members are as contained in the European Patent Office EDP file on
The European Patent Office is in no way liable for these particulars which are merely given for the purpose of information.

19-12-2016

| Patent document cited in search report | | Publication date | Patent family member(s) | | Publication date |
|---|---|---|---|---|---|
| US 2013156280 | A1 | 20-06-2013 | EP | 2577604 A1 | 10-04-2013 |
| | | | GB | 2480864 A | 07-12-2011 |
| | | | US | 2013156280 A1 | 20-06-2013 |
| | | | WO | 2011151448 A1 | 08-12-2011 |
| US 2013317341 | A1 | 28-11-2013 | DE | 102012208625 A1 | 24-12-2013 |
| | | | US | 2013317341 A1 | 28-11-2013 |

EPO FORM P0459

For more details about this annex : see Official Journal of the European Patent Office, No. 12/82